# EUROPEAN PATENT APPLICATION

(11) **EP 1 752 092 A1**
(43) Date of publication of application: **14.02.2007**
(21) Application number: 06016585.9
(22) Date of filing: 08.08.2006
(51) Int. Cl.: A61B 5/022

(54) **Electronic blood pressure monitor**

(30) Priority: 12.08.2005 JP 2005234872
(71) Applicant: Omron Healthcare Co., Ltd., Kyoto 615-0084 (JP)
(72) Inventor: Sawanoi, Yukiya Omron Healthcare Co., Ltd., Kyoto-shi Kyoto 615-0084 (JP); Kishimoto, Hiroshi Omron Healthcare Co., Ltd., Kyoto-shi Kyoto 615-0084 (JP); Matsumura, Naomi Omron Healthcare Co., Ltd., Kyoto-shi Kyoto 615-0084 (JP)
(74) Representative: Kilian, Helmut

(57) **Abstract**

An electronic blood pressure monitor (1) includes a display (2) and a console (4). The console (4) has a plurality of manipulation switches. The display (2) has displaying areas (2B, 2C) associated with the manipulation switches, respectively, for displaying indications provided as the manipulation switches are operated. In operation, the electronic blood pressure monitor (1) displays on display (2) in a predetermined area (2A) a blood pressure value obtained by measuring blood pressure. The console (4) has a user selection switch group (42), a measuring switch (73) and a record recalling switch (74), and these manipulation switches and the displaying areas (2B, 2C) provided on the display (2) and associated with the switches are adjacently arranged, respectively.

## Description

### BACKGROUND OF THE INVENTION

### Field of the Invention

The present invention relates generally to electronic blood pressure monitors and particularly to electronic blood pressure monitors having a function guiding which manipulation switch should be operated.

### Description of the Background Art

Currently, domestic pressure measuring instruments are rapidly increasingly used. Blood pressure is one index employed to analyze circulatory diseases. Analyzing a risk based on blood pressure is effective for example in preventing cerebral apoplexy, cardiac failure, myocardial infarction and other cardiovascular diseases.

Such risk analysis needs to be based on a result of measuring blood pressure over a long period of time of at least one week under a fixed condition. Accordingly, electronic blood pressure monitors have been provided that have a function to record results obtained from measuring blood pressure a plurality of times. Some conventional electronic blood pressure monitors have a function to store results of measuring a plurality of people for blood pressure, a function to store results of measuring blood pressure in the morning, in the evening or for each condition for measurement, and the like.

Japanese Patent Laying-Open No. 2002-272686 discloses an electronic blood pressure monitor shared by a plurality of users. More specifically, when a button pressed to switch users is pressed to designate a user, the blood pressure monitor stores a value in blood pressure as measured of the designated user to a memory area allocated for the designated user.

A conventional blood pressure monitor, for example a blood pressure monitor capable of storing obtained measurements of a plurality of people, allows a user to operate a switch before or after measurement to designate a person to be measured. Similarly, a blood pressure monitor that stores an obtained measurement for each condition for measurement also allows a user to operate a switch before or after measurement to designate a condition for measurement. In these approaches a liquid crystal display displays a result of operating the switch, e.g., a selected user or a selected condition for measurement. However, the display and the switch are each independent and remote from each other. As a result the user has difficulty in associating what is displayed with the operated switch by intuition. While for example a blood pressure monitor capable of setting a plurality of users that has a switch operated to selectively designate each user and stores a value in blood pressure for the user over a long period of time should allow the value to be used to provide an effective disease risk analysis for the user. If a user incorrectly selects another user and without noticing it conducts measurement and thus obtains and stores data over a low period of time, however, the data cannot be used effectively for risk analysis.

For example Japanese Patent Laying-Open No. 2002-272686 describes that when a button is pressed to indicate that users should be switched to designate a user, the designated user's identification symbol is displayed. However, as the positional relationship between the manner of displaying the identification symbol and the button is not considered, the identification symbol is displayed at an area remote from the button or that is not associated with the button. As such, the user readily overlooks the identification symbol and would measure blood pressure without noticing that he/she has performed an erroneous user selecting operation.

### SUMMARY OF THE INVENTION

The present invention contemplates an electronic blood pressure monitor capable of preventing erroneous operation.

In order to achieve the above object the present invention in one aspect provides an electronic blood pressure monitor including: a cuff attached at a site for measurement of blood pressure; a pressure adjustment unit adjusting a pressure applied to the cuff; a pressure detection unit detecting a pressure internal to the cuff; a blood pressure calculation unit calculating a blood pressure value based on a detection signal output from the pressure detection unit; a storage portion storing the blood pressure value thus calculated; a display at least displaying the blood pressure value; and a plurality of manipulation portions, wherein the display includes manipulation displaying portions associated with the plurality of manipulation portions, respectively, for displaying information provided as the manipulation portions are operated, and the manipulation portions and the manipulation displaying portions located on the display and associated with the manipulation portions are adjacently arranged.

This allows a user to confirm at an area adjacent to a manipulation portion the information provided as the manipulation portion is operated. This ensures that the subject notices erroneous operation, and also allows the subject to notice it immediately. This in turn helps to prevent the erroneous operation and ensures that the erroneous operation is prevented. This can provide a more reliable record of blood pressure measured over a long period of time and hence data effective in disease risk analysis.

The present invention in another aspect provides an electronic blood pressure monitor including: a cuff attached at a site for measurement of blood pressure; a pressure adjustment unit adjusting a pressure applied to the cuff; a pressure detection unit detecting a pressure internal to the cuff; a blood pressure calculation unit calculating a blood pressure value based on a detection signal output from the pressure detection unit; a storage portion storing the blood pressure value thus calculated; a display at least displaying the blood pressure value; and a plurality of manipulation portions, wherein the display includes manipulation displaying portions associated with the plurality of manipulation portions, respectively, for displaying information provided as the manipulation portions are operated, and the manipulation displaying portions corresponding to the plurality of manipulation portions, respectively, are arranged to match how the plurality of manipulation portions are arranged.

This allows a user to confirm at a area matching how a manipulation portion is arranged the information provided as the manipulation portion is operated. This ensures that the subject notices erroneous operation, and also allows the subject to notice it immediately. This in turn helps to prevent the erroneous operation and ensures that the erroneous operation is prevented.

The present invention in still another aspect provides an electronic blood pressure monitor including: a cuff attached at a site for measurement of blood pressure; a pressure adjustment unit adjusting a pressure applied to the cuff; a pressure detection unit detecting a pressure internal to the cuff; a blood pressure calculation unit calculating a blood pressure value based on a detection signal output from the pressure detection unit; a storage portion storing the blood pressure value thus calculated; a display at least displaying the blood pressure value; and a plurality of manipulation portions, wherein the display includes manipulation displaying portions associated with the plurality of manipulation portions, respectively, for displaying information provided as the manipulation portions are operated, and for each type of operation, the manipulation portion operated to perform the operation and the manipulation displaying portion associated with the manipulation portion are arranged in a section.

This allows a user to confirm for each type of operation at an area according to the same section as a manipulation portion the information provided as the manipulation portion is operated. This ensures that the subject notices erroneous operation, and also allows the subject to notice it immediately. This in turn helps to prevent the erroneous operation and ensures that the erroneous operation is prevented.

The above described electronic blood pressure monitor has the manipulation displaying portion in the display. This allows the manipulation displaying portion to be configured utilizing a portion that displays a blood pressure value. The electronic blood pressure monitor can be less complicated in structure and thus more inexpensive than having a separate displaying area for the manipulation displaying portion. Furthermore, such electronic blood pressure monitor can be less prone to failure.

Preferably the electronic blood pressure monitor is shared by a plurality of subjects, wherein the plurality of manipulation portions include a manipulation portion operated to indicate which one of the subjects is indicated. This ensures that a subject having performed an operation notices whether the operation erroneously indicates a different subject, and also allows the subject to notice it immediately.

Preferably the electronic blood pressure monitor has a mode of operation including a blood pressure measuring mode and a recalling mode allowing the blood pressure value to be read from the storage portion and displayed, wherein the plurality of manipulation portions include a manipulation portion operated to designate one of the blood pressure measuring mode and the recalling mode as the mode of operation. This ensures that a subject having performed an operation to designate a mode of operation of the electronic blood pressure monitor notices whether he/she designates the mode of operation erroneously or appropriately, and also allows the user to notice it immediately.

Preferably in the storage portion the blood pressure value calculated in blood pressure measurement is stored in association with a condition for the blood pressure measurement and the plurality of manipulation portions include a manipulation portion operated to read from the storage portion the blood pressure value associated with the condition as desired and display the blood pressure value thus read. This ensures that a subject having performed an operation to indicate a condition for measurement of a blood pressure value to be recalled notices whether he/she indicates the condition erroneously or appropriately, and also allows the subject to notice it after the operation immediately.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Figs. 1-3 show in appearance an electronic blood pressure monitor in embodiment.
Fig. 4 shows a configuration in function of the electronic blood pressure monitor in embodiment.
Fig. 5 shows an example of contents of a memory in embodiment.
Fig. 6 is a flow chart of a process performed by the electronic blood pressure monitor in embodiment.
Figs. 7-11 show examples of manners, respectively, of displaying in embodiment.
Figs. 12 and 13 shows another and still another examples, respectively, of a displaying area in embodiment.

### DESCRIPTION OF THE PREFERRED EMBODIMENT

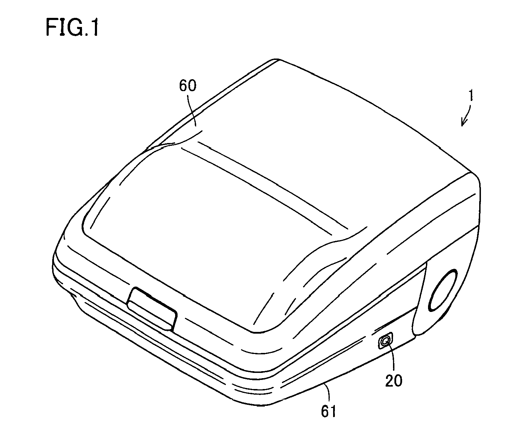

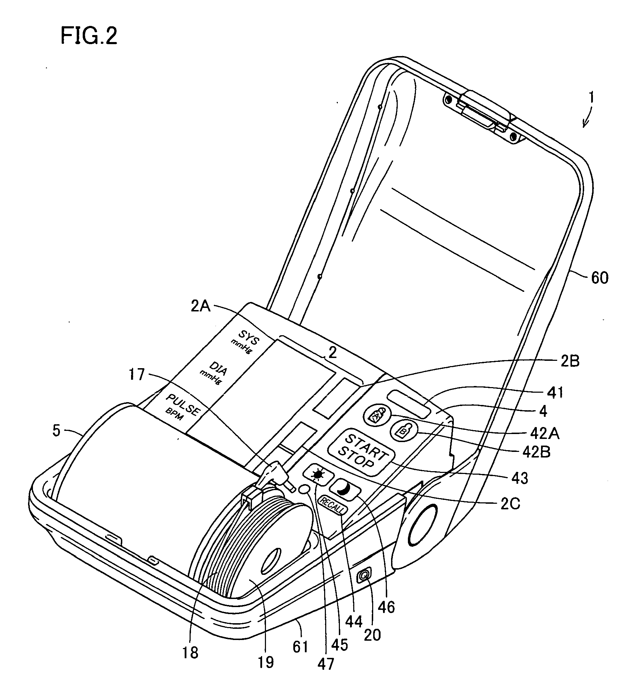

Figs. 1-3 show a configuration of an electronic blood pressure monitor 1 in an embodiment that can be shared by a plurality subjects. Electronic blood pressure monitor 1 includes a main body 61 and a front cover 60 provided to cover a surface of main body 61 for protection. Front cover 60 is provided to be opened and closed, as desired, as shown in Figs. 1 and 2. Electronic blood pressure monitor 1 has modes of operation including a blood pressure monitor measuring mode and a record recalling mode recalling an obtained blood pressure measurement from memory. "Recall" as referred to herein indicates reading data such as an obtained blood pressure measurement from memory and displaying the read data.

Front cover 60 may be formed of transparent material allowing a surface of main body 61 to be externally observable with front cover 60 closed as shown in Fig. 1, or may be formed of opaque material. Front cover 60 formed of transparent material is convenient for conforming what (i.e., blood pressure being measured) is displayed on a display 2 when blood pressure is measured with front cover 60 closed.

Furthermore, main body 61 includes a connector portion 20 located in a side surface of the main body for feeding electronic blood pressure monitor 1 with power, and an air connector 31 (not shown) located at an opposite side surface for supplying/exhausting air in measuring blood pressure.

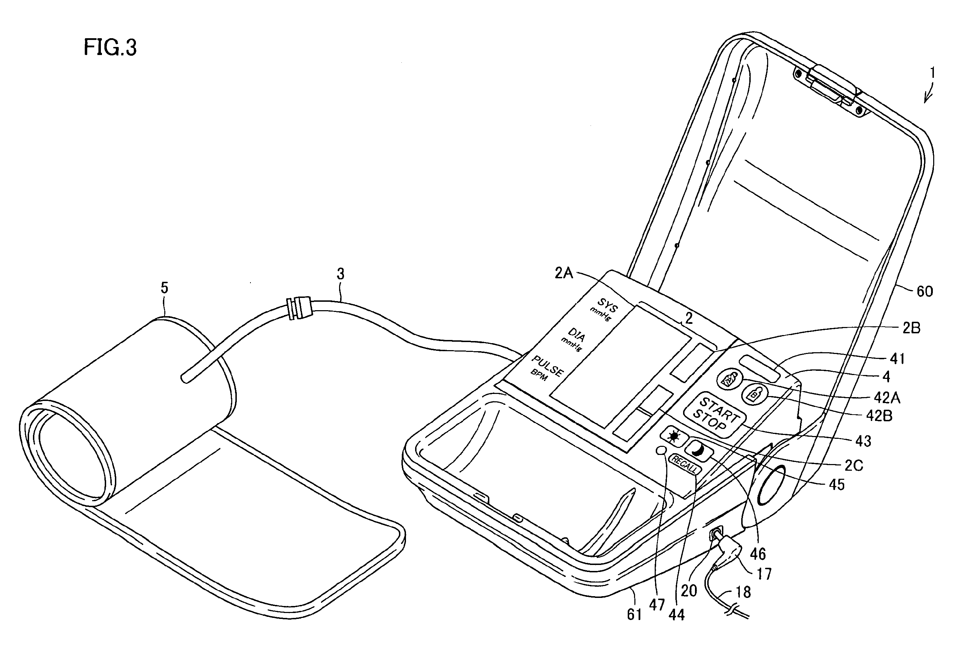

Fig. 3 shows a manner for use of electronic blood pressure monitor 1 in measuring blood pressure. To measure blood pressure, a subject opens front cover 60 (see Fig. 2) and extracts from a front portion of main body 61 a cord reel 19 having a power supply cord 18 would therearound, and a cuff 5. Then, as shown in Fig. 3, power supply cord 18 is unwound from cord reel 19, and a plug 17 of one end of power supply cord 18 is plugged into connector portion 20 and a plug (not shown) of the other end of power supply cord 18 of cord reel 19 is inserted into a plug socket of an alternate current (ac) power supply (a commercial power supply) (not shown). Electronic blood pressure monitor 1 is thus supplied with power from the ac power supply through power supply cord 18.

Cuff 5 is connected to an air tube 3 having one end connected to an air bag (not shown) internal to cuff 5 and the other end inserted into air connector 31 (not shown) of main body 61. Thus through air tube 3 main body 61 can supply cuff 5 (or the air bag) with air and exhaust air from cuff 5.

Electronic blood pressure monitor 1 has cuff 5 and power supply cord 18 detachably attachable to main body 61 and hence separate from main body 61. Alternatively, cuff 5 alone may be provided as a separate component and main body 61 and power supply cord 18 may integrally be provided. Alternatively, power supply cord 18 alone may be provided as a separate component and main body 61 and cuff 5 may integrally be provided. Alternatively, power supply cord 18, cuff 5 and main body 61 may integrally be provided. Furthermore, front cover 60 provided to cover a front surface of main body 61 entirely may alternatively be provided to cover only a portion of the front surface portion excluding that accommodating cord reel 19 having power supply cord 18 wound therearound and cuff 5.

Main body 61 has a surface (a front surface portion) provided with a console 4 operable by a user and display 2 displaying an obtained measurement or the like to allow the user to confirm what is displayed.

Console 4 has a plurality of switches externally operated to receive a variety of instructions. More specifically, console 4 includes a power supply switch 41, a user selection switch group 42 configured of user selection switches 42A and 42B, a measuring switch 43 operated to cause electronic blood pressure monitor 1 to transition to the blood pressure measuring mode and indicate that measurement starts or stops, a record recalling switch 44 operated to instruct electronic blood pressure monitor 1 to transition to the record recalling mode, a morning switch 45 and an evening switch 46 operated to indicate whether data to be recalled is a measurement obtained in the morning or that obtained in the evening, a clock setting switch 47 operated to set a time counted by a timer 14 as described later. The record recalling mode indicates a mode of operation in which the data of an obtained blood pressure measurement stored in a memory 13, which will be described later, internal to main body 61 is read from memory 13 and displayed on display 2.

Herein, as shown in Fig. 3, for each type (or item) of operation, i.e., for each type of instruction received through the operation, a switch provided in console 4 for that operation and an indication displayed on display 2 in response to that switch being operated are arranged in a section. More specifically, display 2 has areas 2A, 2B and 2C. Areas 2B and 2C are displaying portions each displaying an indication in response to a corresponding switch of console 4 being operated. Area 2A displays data of an obtained measurement, such as blood pressure, pulse, measurement time, and the like. Area 2B displays data indicating different users (or subjects) in response to user selection switches 42A and 42B being operated. Area 2C displays content(s) associated with data indicated in areas 2A and 2B. Area 2C for example displays a type of mode of operation of electronic blood pressure monitor 1 that is indicated in response to measuring switch 43 or record recalling switch 44 being operated, and data (e.g., a character, a picture, graphics) indicating "morning" or "evening" in response to morning switch 45 or evening switch 46 being operated. Electronic blood pressure monitor 1 has modes of operation including a "measuring" mode and a "recording" mode. It is assumed that data for displaying in areas 2B and 2C are previously stored for example in memory 13, which will be described later, and read by a central processing unit (CPU) 16, which will be described later, and displayed.

In Fig. 3, areas 2A, 2B and 2C of display 2 displaying data in response to each type of switch being operated, are provided adjacent to a variety of switches, respectively, associated therewith.

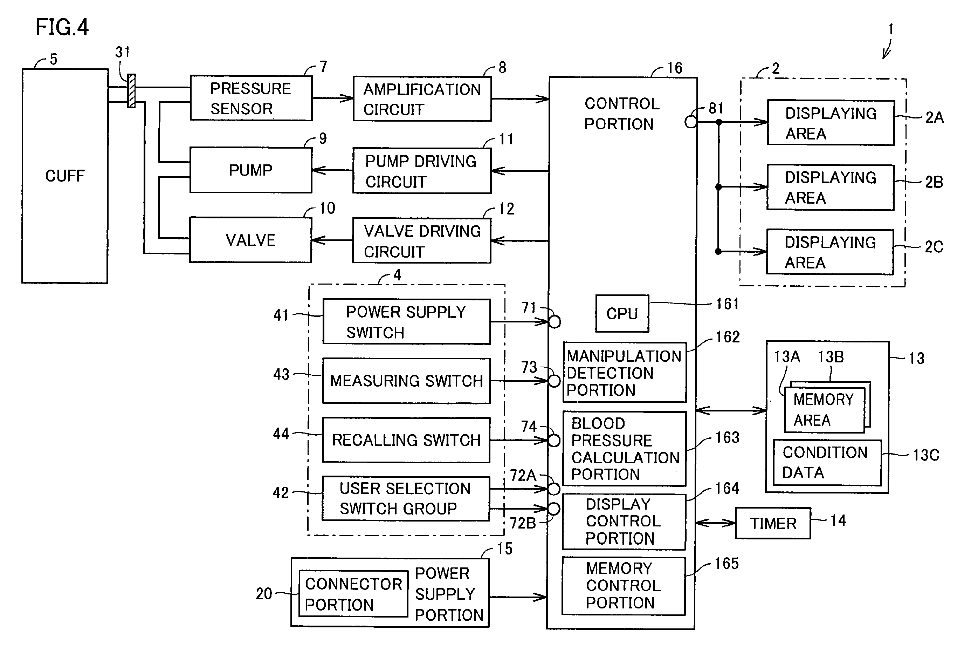

With reference to Fig. 4 the present embodiment provides electronic blood pressure monitor 1 including cuff 5 attached on a subject at a site (an upper arm) for measurement of blood pressure for applying pressure by air pressure, and air tube 3 connecting main body 61 and cuff 5 together. In Fig. 4, a portion rightward of air connector 31 indicates each component corresponding to main body 61. Main body 61 includes display 2 provided to allow a subject to confirm what is displayed, and console 4 provided to be operable externally by the subject. Herein the site for measurement is an upper arm, however it is not limited thereto, and it may be a wrist or a finger.

Main body 61 further includes a pressure sensor 7 outputting in the form of a pulse wave signal a variation in pulse pressure detected at a site for measurement via the air bag (not shown) incorporated in cuff 5, an amplification circuit 8 amplifying a voltage signal indicative of a pressure output from pressure sensor 7, a pump 9 and a valve 10 for adjusting in level a pressure (an air pressure) applied by the air bag to the site for measurement, a pump driving circuit 11 for driving pump 9, a valve driving circuit 12 adjusting how valve 10 is opened/closed, memory 13, timer 14 operating to count time and outputting data of time counted, a power supply portion 15 having connector portion 20 to supply each component of main body 61 with power provided through connector portion 20, and a control portion 16. Control portion 16 has a central processing unit (CPU) 161 controlling each component of main body 61, and input ports 71, 72A, 72B, 73 and 74. Furthermore, control portion 16 includes a manipulation detection portion 162, a blood pressure calculation portion 163, a display control portion 164 and a memory control portion 165 operating as controlled by CPU 161. Manipulation detection portion 162 detects (or determines) which type of switch of console 4 has been operated or whether any switch has been operated. Blood pressure calculation portion 163 calculates blood pressure and pulse. Display control portion 164 controls display 2 to operate for displaying. Memory control portion 165 has a function to access memory 13. The functions of manipulation detection portion 162, blood pressure calculation portion 163, display control portion 164 and memory control portion 165 are implemented by CPU 161 reading and executing a predetermined program previously stored in memory 13. while each portion's function is herein implemented by a program, the entirety or a portion thereof may be implemented by a circuit or other similar hardware.

Although in Fig. 4 at console 4 clock setting switch 47 is not shown, when clock setting switch 47 is operated CPU 161 responsively sets time count data of timer 14 based on how specifically clock setting switch 47 is operated.

Power supply 15 has a rechargeable battery (not shown). When connector portion 20 does not have power supply cable 18 connected thereto, power supply portion 15 supplies power from the battery to control portion 16 including CPU 161. When connector portion 20 has power supply cable 18 connected thereto, through power supply cable 18 an external ac power supply supplies power, which is supplied by power supply portion 15 to each component of main body 61 and also used to charge the battery. Note that power supply portion 15 having the rechargeable battery may alternatively be implemented by an alkaline battery. In that case, while connector portion 20 does not have power supply cable 18 connected thereto, the alkaline battery supplies power to control portion 16 including CPU 161. When connector portion 20 has power supply cable 18 connected thereto, through power supply cable 18 an external ac power supply supplies power, which is supplied by power supply portion 15 to each component of main body 61, and the alkaline battery is electrically disconnected from each component of main body 61.

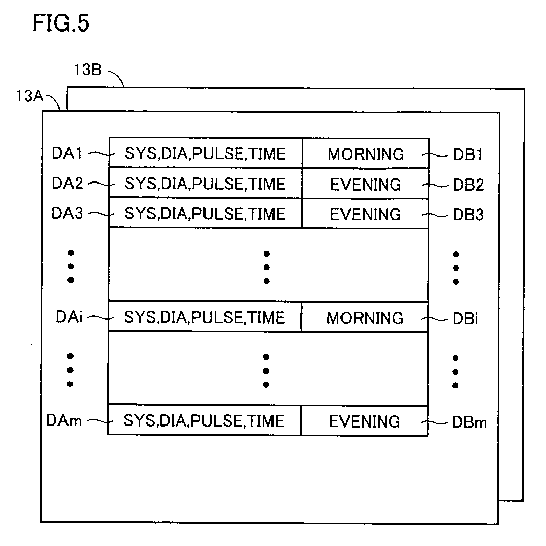

Memory 13 has an individual memory area for an obtained measurement for each user to allow electronic blood pressure monitor 1 to be shared by a plurality of people. Herein, electronic blood pressure monitor 1 is shared by two persons for the sake of illustration. Accordingly, memory 13 has areas 13A and 13B each for storing an obtained measurement for a user (or a subject). As shown in Fig. 5, memory areas 13A and 13B each store data DAi indicating an obtained blood pressure measurement and data DBi indicating a condition for measurement such that data DAi and DBi are associated with each other, wherein i = 1, 2, 3, ..., i, ..., m. Data DAi includes data of systolic blood pressure (mmHg) indicated in the figure as "SYS", diastolic blood pressure (mmHg) indicated in the figure as "DIA", a pulse rate per minute indicated in the figure as "PULSE", and measurement time indicated in the figure as "TIME". For example, data DBi indicates whether a subject measured blood pressure in the morning or in the evening. Alternatively, the data indicates whether a subject measured blood pressure after getting up or before going to bed. Alternatively, the data indicates whether a subject measured blood pressure before or after the subject had drug administration. Alternatively, the data indicates whether a subject measured blood pressure before or after exercise. Alternatively, the data indicates an weekend or a week day. Herein, by way of example, morning/evening is assumed as a condition for measurement.

The condition for measurement "morning/evening" is temporarily stored in memory 13 as condition data 13C. If CPU 161 determines that measurement time data received from timer 14 corresponds to a predetermined period of time corresponding to the morning, condition data 13C is set to indicate "morning", and if CPU 161 determines that measurement time data received from timer 14 corresponds to a predetermined period of time corresponding to the evening, then condition data 13C is set to indicate "evening". Note that the data of the predetermined period of time indicating the morning and that of the predetermined period of time indicating the evening are assumed to be previously stored in memory 13. Thus CPU 161 compares the measurement time data received from timer 14 with the data of the predetermined period of time read from memory 13 to determine a condition for measurement.

With reference to Figs. 3 and 4, console 4 includes power supply switch 41, user selection switches 42A and 42B operated to distinguish subjects, measuring switch 43 operated to indicate whether to start or stop pressurization (or measurement), clock setting switch 47, record recalling switch 44 operated to recall data of an obtained measurement stored (or read data of an obtained measurement from memory 13 and display the read data on display 2), and morning switch 45 and evening switch 46.

Morning switch 45 and evening switch 46 are operated to designate data of an obtained measurement stored that should be to be recalled. More specifically, the switches are operated to designate whether data of a measurement that is obtained in the morning or that of a measurement that is obtained in the evening should be recalled.

When user selection switch 42A or 42B is operated, in response to which switch having been operated, either memory area 13A or 13B is designated as a memory area that should store data of an obtained measurement or a memory area searched for recalling such data. When user selection switch 42A is operated memory area 13A is designated and when user selection switch 42B is operated memory area 13B is designated.

Power supply switch 41 is operated to turn on/off power supplied from power supply portion 15 to each component (excluding control portion 16) for operation, as will be described later. As such, if, in operation, power supply switch 41 is operated, the operation is stopped (or terminated).

In Fig. 4 at console 4 power supply switch 41, user selection switch group 42 formed of user selection switches 42A and 42B, measuring switch 43 and record recalling switch 44 are shown for the sake of illustration.

As described above, control portion 16 has input ports 71-74. Input port 71 is connected to power supply switch 41 to receive a signal output as power supply switch 41 is operated. Input ports 72A and 72B are connected to user selection switches 42A and 42B, respectively, to receive signals output as the switches are operated. Input port 73 is connected to measuring switch 43 to receive a signal output as measuring switch 43 is operated. Input port 74 is connected to record recalling switch 44 to receive a signal output as record recalling switch 44 is operated. The signal received at each input port is detected by CPU 161.

Input ports 71-74 each receive a signal in the form of a pulse signal having a width of a predetermined period of time. More specifically, when a corresponding switch is operated, the pulse signal transitions from "0" to "1" and thereafter for only a short, predetermined period of time holds the level of "1" and transitions to "0" after that predetermined period of time elapses. If CPU 161 detects that any input port receives the pulse signal, CPU 161 determines (or detects) that the switch connected to that port has been operated.

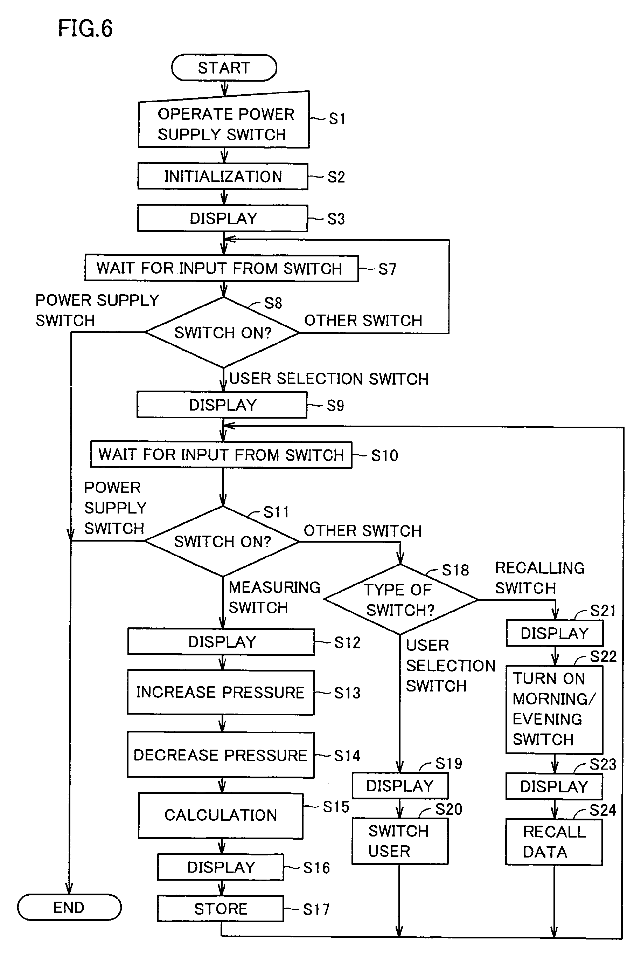

Fig. 6 is a flowchart for illustrating how in the present embodiment electronic blood pressure monitor 1 operates (to measure blood pressure and recall data of a measurement). The Fig. 6 flowchart is stored previously as a program in memory 13 at a predetermined area and read and executed by CPU 161.

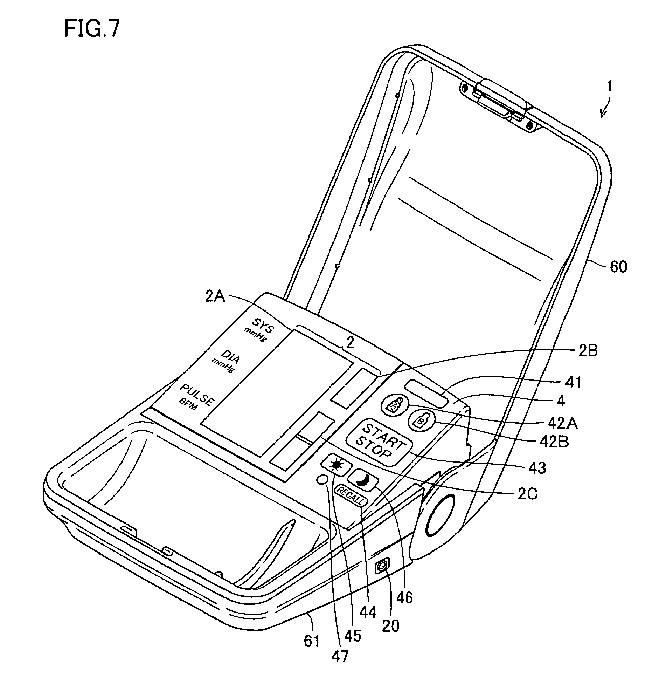

The Fig. 6 procedure is followed and Figs. 7-11 are referenced to describe the operation. Note that it is assumed that to measuring blood pressure a subject connects air tube 3 to air connector 31 and has cuff 5 wound around a site for measurement. Furthermore it is assumed that the subject inserts plug 17 into connector portion 20 and connects power supply cable 18 to ac power supply to allow main body 61 to have each component with power supplied thereto. In recalling blood pressure measurement data, the subject inserts plug 17 into connector portion 20 and connects power supply cable 18 to the ac power supply. However, it is not necessary to attach cuff 5. As such, while the subject may connect air tube 3 to air connector 31, the subject may not wind cuff 5 around the site for measurement.

In Figs. 7-11, in reality, as shown in Fig. 3, main body 61 has cuff 5 connected thereto and power supply cord 18 is connected. However, in order to provided a simplified diagrammatical representation, these connections are not shown.

In such condition the subject prepares electronic blood pressure monitor 1 and opens main cover 60, as shown in Fig. 7, when display 2 does not display any indication, and the subject operates power supply switch 41 to supply each component of electronic blood pressure monitor 1 with power to start it to operate (step(S)1). Manipulation detection portion 162 detects, as based on a level of a signal received at input port 71, that power supply switch 41 has been operated. In response to such detection, CPU 161 initializes a working memory (not shown) of control portion 16 and controls each component to exhaust air from the air bag of cuff 5 to effect correction to allow pressure sensor 7 to provide an output of 0 mmHg (S2).

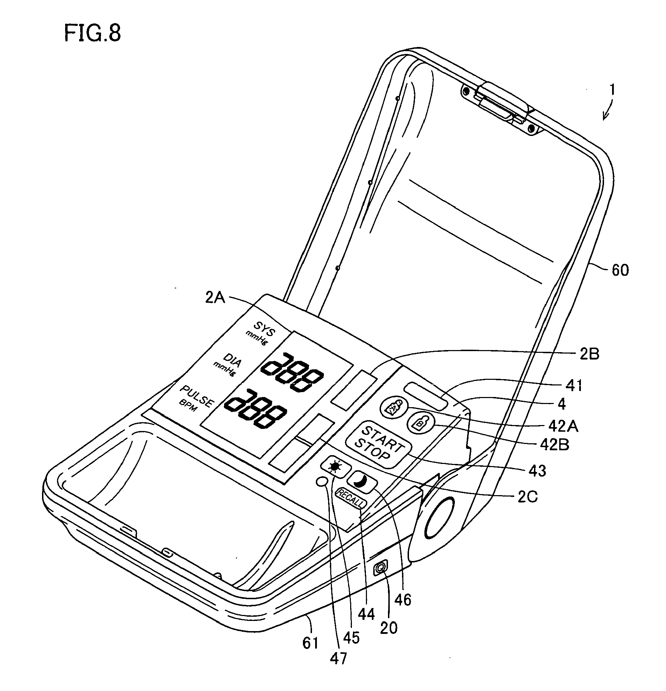

Subsequently, display control portion 164 causes display 2 to display data, as shown in Fig. 8, in area 2A to inform the subject that power supply is turned on and each component is supplied with power to be capable of operation (to measure blood pressure or recall data of a measurement) (S3). The subject confirming that indication can confirm that in response to power supply switch 41 having been operated, each component is supplied with power (S3).

Subsequently CPU 161 transitions to a state and therein waits until there is an input received from a switch (S7) until manipulation detection portion 162 detects that any switch is operated (S8). In that state, manipulation detection portion 162 detects that the subject has operated any switch, as based on which of input ports 71-74 receives a signal varying in level (S8). While manipulation detection portion 162 makes a decision, as based on the fact that an input port receives a signal varying in level, that a switch other than user section switches 42A and 42B and power supply switch 41 is operated ("other switch" at S8), CPU 161 assumes the state in which it waits until there is an input received from a switch (S7).

If manipulation detection portion 162 determines that input port 71 corresponding to power supply switch 41 receives a pulse signal ("power supply switch at S8"), CPU 161 follows the decision and terminates a series of operations. Thus the current process is terminated.

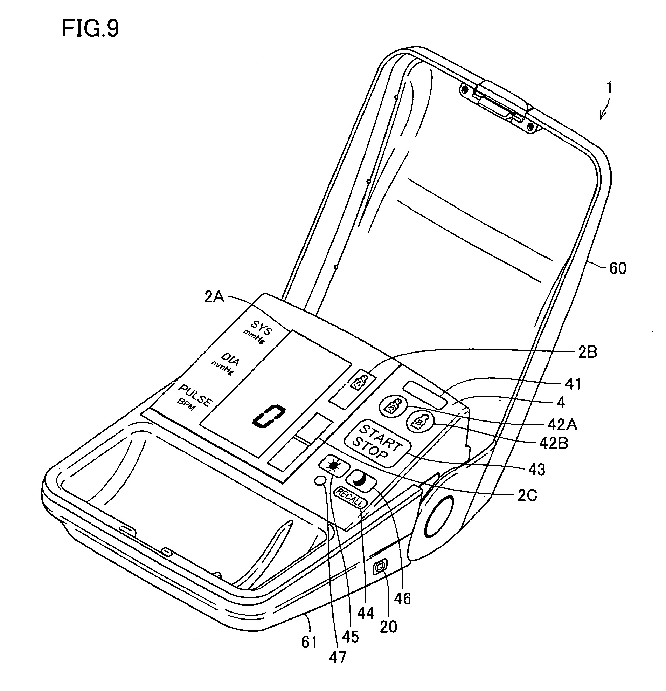

If manipulation detection portion 162 determines that input port 72A or 72B receives a pulse signal ("user selection switch" at S8), an operation is performed such as follows: more specifically, if a decision is made that input port 72A receives a pulse signal then in accordance with the decision memory control portion 165 designates the user as "A", i.e., designates memory area 13A as an area at which an obtained measurement is written or an area from which an obtained measurement is recalled. If a decision is made that input port 72B receives a pulse signal, then in accordance with the decision memory control portion 165 designates the user as "B", i.e., designates memory area 13B as an area to which an obtained measurement is written or an area from which an obtained measurement is recalled. Display control portion 164 controls display 2 to display a result of a decision made by manipulation detection portion 162, i.e., a result of designating a memory area to be that to which an obtained measurement is written and that from which an obtained measurement is recalled, such as shown in Fig. 9 (S9).

In Fig. 9 display control portion 164 controls display 2 to display "0" in area 2A to indicate that the blood pressure monitor is now ready to operate to measure blood pressure or recall data of an obtained measurement. Furthermore, display control portion 164 controls display 2 to display in area 2B an icon, such as a picture, graphics, a character or the like, indicating user "A" to explicitly indicate that as a user, "A" has been selected. This can inform the subject that in electronic blood pressure monitor 1 a setting of selecting a user is correctly done and that electronic blood pressure monitor 1 is in a state capable of operation.

Subsequently CPU 161 transition to the state in which it waits until there is an input received from a switch (S10). This state continues until manipulation detection portion 162 detects that any input port receives a pulse signal. If any input port receives a pulse signal, manipulation detection portion 162 determines to which switch the pulse signal corresponds (S11). This decision is made from which input port receives the pulse signal. If a decision is made that the pulse signal is received at input port 71 (power supply switch" at S11) then in accordance with the decision CPU 161 instructs power supply portion 15 to stop power supplied to each component. Supplying power by power supply portion 15 to each component thus stops. Accordingly, as shown in Fig. 7, an indication also disappears, and a series of operations ends.

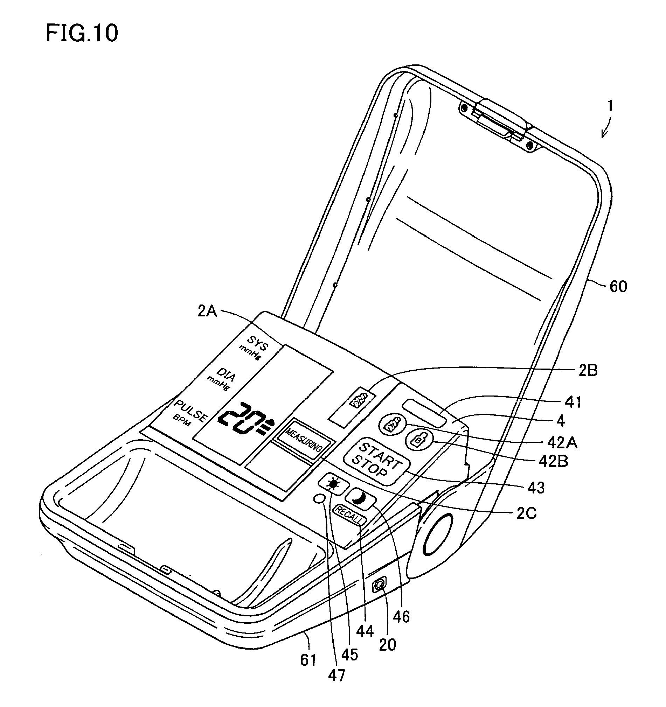

If a decision is made that input port 73 receives a pulse signal ("measuring switch" at S 11) then in accordance with the decision CPU 161 starts a process involving displaying data, as shown in Fig. 10, for measuring blood pressure, as indicated in S12 et. seq., as described later.

If an input port other than input ports 71 and 73 receives a pulse signal, then manipulation detection portion 162 determines that a switch other than measuring switch 43 and power supply switch 41 has been operated ("other switch" at S11) and the type of the switch operated is determined (S 18). More specifically, if the input port having received the pulse signal is input port 72A or 72B, a decision is made that the user (or subject) that has been set, as determined in S8, as a switch was operated, is switched ("user selection switch" at S 18). In accordance with the decision, display control portion 164 switches an indication of a user in displaying area 2B to an indication of a user corresponding to the input port receiving the pulse signal (S 19).

Subsequently memory control portion 165 follows the decision made by manipulation detection portion 162 to switch a setting for the user (S20). For example if input port 72A receives a pulse signal the user is set as "A", i.e., memory area 13A is set as an area to which an obtained measurement is written and an area from which an obtained measurement is recalled. If input port 72B receives a pulse signal the user is set as "B", i.e., memory area 13B is set as an area to which an obtained measurement is written and an area from which an obtained measurement is recalled. Thereafter, CPU 161 returns to S10 and transitions to the state in which it waits until there is an input received from a switch.

In contrast, if a decision is made that a pulse signal is received at input port 74 ("record recalling switch" at S 18), then in accordance with the decision display control portion 164 displays the characters "record" in displaying area 2C to explicitly indicate that the record recalling mode is set to recall data of an obtained measurement recorded (S21). Subsequently the subject operates morning switch 45 or evening switch 46 to designate which of data of a measurement obtained in the morning or that obtained in the evening should be recalled (S22). At that time, manipulation detection portion 162 determines which switch has been operated. In accordance with the decision display control portion 164 determines which of the data of the measurement obtained in the morning or that obtained in the evening is recalled up to displaying area 2A and that decision is additionally displayed in displaying area 2C (S23).

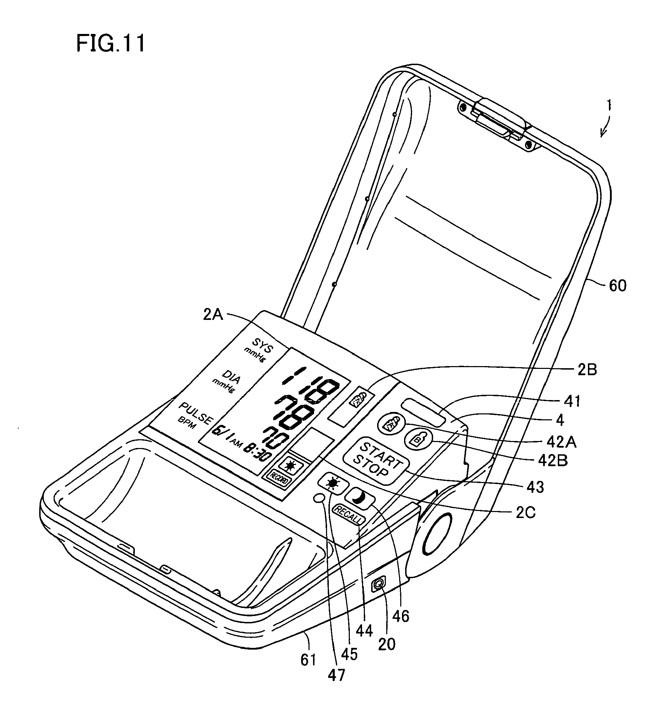

Subsequently memory control portion 165 reads an obtained blood pressure measurement from memory 13 and the read measurement is displayed by display control portion 164 in displaying area 2A (S24). More specifically, memory control portion 165 reads, from one of memory areas 13A and 13B corresponding to users "A" or "B", respectively, set in S8 or S29, data DAi indicating an obtained measurement that corresponds to data DBi indicating a condition for measurement that indicates "morning" or "evening" designated in S22, and display control portion 164 displays the read data of the obtained measurement in displaying area 2A in a manner as shown in Fig. 11 by way of example. In Fig. 11, displaying area 2C displays the characters "record" and a picture of the sun indicating "morning". Thereafter, the control returns to step S 10.

A blood pressure measurement operation performed in S12 et. seq. will now be described.

As the subject operates measuring switch 43 ("measuring switch" at S 11), CPU 161 transitions to the blood pressure measuring mode. Accordingly, display control portion 164 displays the characters "measuring" in displaying area 2C (S12). This can inform the subject that the instrument has transitioned to state ready for measuring blood pressure, as instructed.

Subsequently CPU 161 controls each component to increase the pressure internal to the air bag gradually to the subject's systolic blood pressure or approximately + 40 mmHg (S13) and thereafter gradually decreases the pressure internal to the air bag (S14). In this pressure reduction process the pressure internal to the air bag is detected by pressure sensor 7 and amplification circuit 8, and blood pressure calculation portion 163 receives a voltage signal from amplification circuit 8 and from the received voltage signal calculates a blood pressure (systolic blood pressure and diastolic blood pressure) value and a pulse rate and stores them to CPU 161 at a memory internal thereto (not shown) temporarily (S 15). Display control portion 164 controls display 2 to display the calculated blood pressure value and pulse rate in displaying area 2A (S 16). The process for increasing and decreasing pressure for measuring blood pressure is similar to that performed by a conventional electronic blood pressure monitor. Note that while herein the blood pressure monitor measures blood pressure in the pressure reduction process, it may do so in the pressurization process. In measuring blood pressure, the blood pressure monitor detects (or calculates) a blood pressure value from moment to moment, and accordingly updates and thus displays the value in displaying area 2A, as shown in Fig. 10.

When CPU 161 completes calculating and displaying blood pressure and pulse rate, CPU 161 obtains measurement time data based on time count data provided from timer 14. If the obtained time count data corresponds to a predetermined period of time corresponding to "morning", condition data 13C is set as "morning". If the obtained time count data corresponds to a predetermined period of time corresponding to "evening" then condition data 13C is set as "evening". Then the obtained measurement time data, condition data 13C read from memory 13, and data indicating the value in blood pressure (systolic blood pressure and diastolic blood pressure) and pulse rate read from the internal memory of CPU 161 are associated with each other. Memory control portion 165 stores the associated data as data DAi of an obtained measurement and data DBi of a condition for measurement to memory area 13A or 13B set in S8 or S20 as an area at which the data are written (or stored) (S17). Thus a blood pressure measurement ends and the control returns to S10.

Data provided as a variety of switches is operated in S3, S9, S12, S 19, S21 and S23 are thus displayed in areas 2A, 2B and 2C provided adjacent to the variety of switches operated. This helps a subject to confirm an instruction provided to electronic blood pressure monitor 1 by operating a switch, or an operation of electronic blood pressure monitor 1 that is set by an instruction. Thus the subject can immediately notice erroneous operation, and this can help to prevent erroneous operation.

### Displaying Area in Another Example

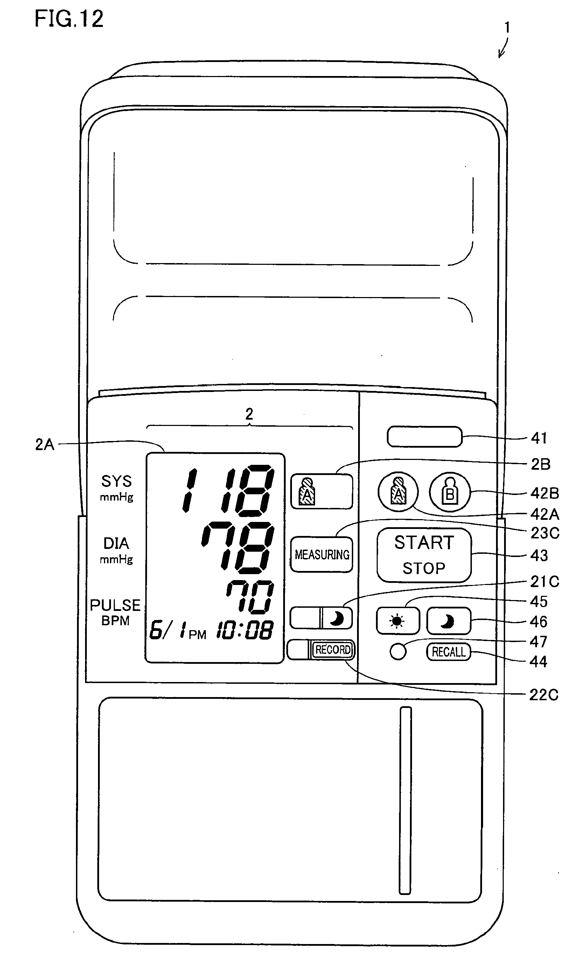

With reference to Fig. 12, display 2 has a displaying area in another exemplary arrangement, as will now be described hereinafter. Herein, an area for indicating information provided as each manipulation switch is operated is arranged to match how the manipulation switch is arranged.

In Fig. 12, areas 2A, 2B, 21C, 22C and 23C displaying data provided as a variety of switches is operated are arranged to match how the variety of switches is arranged. Area 2B displays in the same arrangement as user selection switches 42A and 42B adjacent thereto the data provided as the switches are operated. In Fig. 12, user selection witch 42A located on the left hand has been operated. Accordingly, displaying area 2B also displays the letter "A" on the left hand.

Furthermore, area 21C displays in the same arrangement as morning switch 45 and evening switch 46 adjacent thereto the data provided as the switches are operated. In Fig. 12, evening switch 46 located on the right hand has been operated. Accordingly, displaying area 21C also displays a picture of the moon on the right hand.

Furthermore area 22C displays data provided as record recalling switch 44 adjacent thereto is operated. When record recalling switch 44 is operated, the characters "record" are displayed in area 22C on the right hand.

Furthermore area 23C displays the characters "measuring" when measuring switch 43 adjacent to the area is operated.

### Displaying Area in Still Another Example

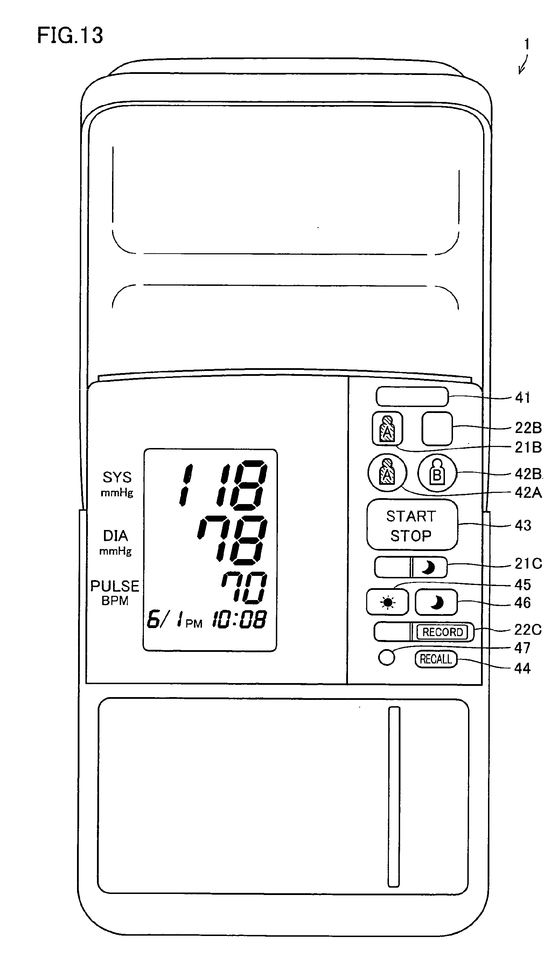

With reference to Fig. 13 display 2 has a displaying area in still another exemplary arrangement, as will now be described hereinafter.

Fig. 13 shows displaying area 2B more adjacent to user selections witches 42A and 42B. Displaying area 2B has areas 21B and 22B provided in the same arrangement as switches 42A and 42B and displaying data provided as the switches are operated. In Fig. 13, user selection witch 42A located on the left hand has been operated. Accordingly, displaying area 21B located on the left hand displays the letter "A".

While the present embodiment assumes that a plurality of people share electronic blood pressure monitor 1, it is also applicable if the blood pressure monitor cannot be shared, i.e., if memory 13 does not have a memory area for each individual to store an obtained measurement. In that case, console 4 does not have user selection switch group 42 and display 2 does not have displaying area 2B.

Furthermore displaying area 2C or 22C can display the record recalling mode/the blood pressure measuring mode to inform a subject of the current mode of operation of electronic blood pressure monitor 1.

Herein when data of an obtained blood pressure measurement is stored to memory 13 or such data is recalled from memory 13, i.e., when the Fig. 6 S 17 or S24 is performed, area 2C displays data in a different manner, e.g., in a different color; such that the area staying illuminated is controlled to flash on and off; or vice versa; or the area has the data erased. This can inform the subject to prohibit the subject from operating a switch. This can prevent an external factor, i.e., a subject from operating a switch while memory 13 is being accessed for data. As such external factor can be eliminated, data corruption and other failures can be prevented that would result from otherwise prevented normal access to memory 13.

Although the present invention has been described and illustrated in detail, it is clearly understood that the same is by way of illustration and example only and is not to be taken by way of limitation, the spirit and scope of the present invention being limited only by the terms of the appended claims.

## Claims

1. An electronic blood pressure monitor comprising:
a cuff (5) attached at a site for measurement of blood pressure;
a pressure adjustment unit (9, 10) adjusting a pressure applied to said cuff;
a pressure detection unit (7, 8) detecting a pressure internal to said cuff;
a blood pressure calculation unit (163) calculating a blood pressure value based on a detection signal output from said pressure detection unit;
a storage unit (13) storing said blood pressure value calculated by said blood pressure calculation unit;
a display (2) at least displaying said blood pressure value; and
a plurality of manipulation portions, wherein
said display includes manipulation displaying portions (2A, 2B, 2C) associated with said plurality of manipulation portions, respectively, for displaying information provided as said manipulation portions are operated, and
said manipulation portions and said manipulation displaying portions located on said display and associated with said manipulation portions are adjacently arranged.

2. The electronic blood pressure monitor according to claim 1, shared by a plurality of subjects, wherein said plurality of manipulation portions include a manipulation portion (42) operated to indicate which one of said subjects is indicated.

3. The electronic blood pressure monitor according to claim 1, having a mode of operation including a blood pressure measuring mode and a recalling mode allowing said blood pressure value to be read from said storage unit and displayed, wherein said plurality of manipulation portions include a manipulation portion (43, 44) operated to designate one of said blood pressure measuring mode and said recalling mode as said mode of operation.

4. The electronic blood pressure monitor according to claim 1, wherein:
in said storage unit said blood pressure value calculated in blood pressure measurement is stored in association with a condition for said blood pressure measurement; and
said plurality of manipulation portions include a manipulation portion operated to read from said storage unit said blood pressure value associated with said condition as desired and display read said blood pressure value.

5. An electronic blood pressure monitor comprising:
a cuff (5) attached at a site for measurement of blood pressure;
a pressure adjustment unit (9, 10) adjusting a pressure applied to said cuff;
a pressure detection unit (7, 8) detecting a pressure internal to said cuff;
a blood pressure calculation unit (163) calculating a blood pressure value based on a detection signal output from said pressure detection unit;
a storage unit (13) storing said blood pressure value calculated by said blood pressure calculation unit;
a display (2) at least displaying said blood pressure value; and
a plurality of manipulation portions, wherein
said display includes manipulation displaying portions (2A, 2B, 2C) associated with said plurality of manipulation portions, respectively, for displaying information provided as said manipulation portions are operated, and
said manipulation displaying portions corresponding to said plurality of manipulation portions, respectively, are arranged to match how said plurality of manipulation portions are arranged.

6. The electronic blood pressure monitor according to claim 5, shared by a plurality of subjects, wherein said plurality of manipulation portions include a manipulation portion (42) operated to indicate which one of said subjects is indicated.

7. The electronic blood pressure monitor according to claim 5, having a mode of operation including a blood pressure measuring mode and a recalling mode allowing said blood pressure value to be read from said storage unit and displayed, wherein said plurality of manipulation portions include a manipulation portion (43, 44) operated to designate one of said blood pressure measuring mode and said recalling mode as said mode of operation.

8. The electronic blood pressure monitor according to claim 5, wherein:
in said storage unit said blood pressure value calculated in blood pressure measurement is stored in association with a condition for said blood pressure measurement; and
said plurality of manipulation portions include a manipulation portion operated to read from said storage unit said blood pressure value associated with said condition as desired and display read said blood pressure value.

9. An electronic blood pressure monitor comprising:
a cuff (5) attached at a site for measurement of blood pressure;
a pressure adjustment unit (9, 10) adjusting a pressure applied to said cuff;
a pressure detection unit (7, 8) detecting a pressure internal to said cuff;
a blood pressure calculation unit (163) calculating a blood pressure value based on a detection signal output from said pressure detection unit;
a storage unit (13) storing said blood pressure value calculated by said blood pressure calculation unit;
a display (2) at least displaying said blood pressure value; and
a plurality of manipulation portions, wherein
said display includes manipulation displaying portions (2A, 2B, 2C) associated with said plurality of manipulation portions, respectively, for displaying information provided as said manipulation portions are operated, and
for each type of operation, said manipulation portion operated to perform said operation and said manipulation displaying portion associated with said manipulation portion are arranged in a section.

10. The electronic blood pressure monitor according to claim 9, shared by a plurality of subjects, wherein said plurality of manipulation portions include a manipulation portion (42) operated to indicate which one of said subjects is indicated.

11. The electronic blood pressure monitor according to claim 9, having a mode of operation including a blood pressure measuring mode and a recalling mode allowing said blood pressure value to be read from said storage unit and displayed, wherein said plurality of manipulation portions include a manipulation portion (43, 44) operated to designate one of said blood pressure measuring mode and said recalling mode as said mode of operation.

12. The electronic blood pressure monitor according to claim 9, wherein:
in said storage unit said blood pressure value calculated in blood pressure measurement is stored in association with a condition for said blood pressure measurement; and
said plurality of manipulation portions include a manipulation portion operated to read from said storage unit said blood pressure value associated with said condition as desired and display read said blood pressure value.

13. A method of displaying in an electronic blood pressure monitor including a cuff (5) attached at a site for measurement of blood pressure,
a pressure adjustment unit (9, 10) adjusting a pressure applied to said cuff,
a pressure detection unit (7, 8) detecting a pressure internal to said cuff,
a blood pressure calculation unit (163) calculating a blood pressure value based on a detection signal output from said pressure detection unit,
a storage unit (13) storing said blood pressure value calculated by said blood pressure calculation unit,
a display (2) at least displaying said blood pressure value, and
a plurality of accepting portions externally operated to receive instruction, the method comprising the steps of:
detecting an accepting portion of said plurality of accepting portions that is externally operated (162, S18); and
controlling displaying information identifying said instruction accepted at said accepting portion detected in the step of detecting, said information being displayed on said display adjacent to said accepting portion detected (164, S19, S21).

14. A method of displaying in an electronic blood pressure monitor including a cuff (5) attached at a site for measurement of blood pressure,
a pressure adjustment unit (9, 10) adjusting a pressure applied to said cuff,
a pressure detection unit (7, 8) detecting a pressure internal to said cuff,
a blood pressure calculation unit (163) calculating a blood pressure value based on a detection signal output from said pressure detection unit,
a storage unit (13) storing said blood pressure value calculated by said blood pressure calculation unit,
a display (2) at least displaying said blood pressure value, and
a plurality of accepting portions externally operated to receive instruction, the method comprising the steps of:
detecting an accepting portion of said plurality of accepting portions that is externally operated (162, S 18); and
controlling displaying identification information identifying said instruction accepted at said accepting portion detected in the step of detecting, said information being displayed on said display (164, S 19, S21), wherein in the step of controlling, said identification information corresponding to each of said accepting portions is displayed on said display at a position according to how said plurality of accepting portions are arranged.

15. A method of displaying in an electronic blood pressure monitor including
a cuff (5) attached at a site for measurement of blood pressure,
a pressure adjustment unit (9, 10) adjusting a pressure applied to said cuff,
a pressure detection unit (7, 8) detecting a pressure internal to said cuff,
a blood pressure calculation unit (163) calculating a blood pressure value based on a detection signal output from said pressure detection unit,
a storage unit (13) storing said blood pressure value calculated by said blood pressure calculation unit,
a display (2) at least displaying said blood pressure value, and
a plurality of accepting portions externally operated to receive instruction, the method comprising the steps of:
detecting an accepting portion of said plurality of accepting portions that is externally operated (162, S18); and
controlling displaying identification information identifying said instruction accepted at said accepting portion detected in the step of detecting, said information being displayed on said display (164, S 19, S21), wherein said identification information of said instruction displayed on said display and said accepting portion having received said instruction are arranged in a section for each type of said instruction.
